# EUROPÄISCHE PATENTSCHRIFT

(11) **EP 1 399 125 B1**
(45) Veröffentlichungstag und Bekanntmachung des Hinweises auf die Patenterteilung: **12.08.2009**
(21) Anmeldenummer: 01971965.7
(22) Anmeldetag: 27.08.2001
(51) Int. Cl.: A61K 8/04, A61K 8/73, A61Q 5/02, C11D 3/50

(54) **PFLEGEMITTEL, ENTHALTEND MATRIXPARTIKEL MIT EINER PARFÜMKOMPONENTE UND EINER WASCHAKTIVEN KOMPONENTE**
CLEANSING/CONDITIONING AGENTS CONTAINING MATRIX PARTICLES WITH A PERFUME COMPONENT AND A WASHING-ACTIVE COMPONENT
AGENTS LAVANTS ET CONDITIONNANTS CONTENANT DES PARTICULES SOUS FORME DE MATRICE AVEC UN PARFUM ET UN AGENT LAVANT

(30) Priorität: 08.09.2000 DE 10044382
(43) Veröffentlichungstag der Anmeldung: 24.03.2004
(73) Patentinhaber: Symrise GmbH & Co. KG, 37603 Holzminden (DE)
(72) Erfinder: TRAGE, Norbert, 51105 Köln (DE); BERTRAM, Ralf, 37603 Holzminden (DE); SONNENBERG, Steffen, 37603 Holzminden (DE); MEYKNECHT, Jürgen, F-75004 Paris (FR)
(74) Vertreter: Eisenführ, Speiser & Partner
(86) Internationale Anmeldenummer: PCT/EP2001/009839
(87) Internationale Veröffentlichungsnummer: WO 2002/019983

(56) Entgegenhaltungen:
- EP-A- 0 334 490
- EP-A- 0 539 025
- WO-A-98/12291
- WO-A-98/15192
- GB-A- 1 367 622
- US-A- 4 563 187
- US-A- 5 686 405
- US-A- 6 024 943

## Beschreibung

Die Erfindung betrifft Pflegemittel, die Matrixpartikel mit einer Parfümkomponente und einer waschaktive Tensidkomponente enthalten, wobei die Matrixpartikel bevorzugt aus einem gelförmigen Polysaccharid bestehen. Die Pflegemittel können beispielsweise in Körperreinigungsmittel und Haushaltsreinigern verwendet werden.

Die Verkapslung von Produkten auf verschiedenen technischen Gebieten (z.B. Arzneimittel, Farbstoffe, Nahrungsmittel usw.), ist bekannt, um Produkte vor einer Anwendung von unerwünschten Einflüssen zu schützen und sie gezielt einzusetzen.

Auf dem Gebiet der Pflegemittel ist es bekannt, Tensidkomponenten und gegebenenfalls auch Parfümkomponenten als Koazervate zu verkapseln. Koazervate bestehen aus eine Schale (z.B. aus Gelatine, Cellulosen, Gummi Arabicum, Polyvinylalkohol und anderen bzw. Kombinationen daraus) und einem Füllgut.

Eine Freisetzung des Füllguts aus dem Koazervat ist nur durch eine gezielte mechanische oder physikalische Zerstörung der Schale möglich. Wünschenswert ist eine leichte mit der Anwendung verbundene Freisetzung des Füllguts.

Bedingt durch Ausfällprozesse ist eine Beladung der Koazervatkapseln mit größeren festen bzw. wasserlöslichen Bestandteilen nicht möglich. Dies ist jedoch für viele Anwendungsformen, z.B. der Aufnahme von Farbpartikeln, erwünscht.

Aufgabe der vorliegenden Erfindung war es, Pflegemittel, insbesondere für die Körperreinigung und technischen Einsatz vor einer Anwendung vor unerwünschter Beeinträchtigung zu schützen, in der Wahl der Inhaltsstoffe weitgehend nicht beschränkt zu sein und eine leichte Freisetzung der Inhaltsstoffe bei der Anwendung zu ermöglichen.

Es wurden Pflegemittel gefunden, die Matrixpartikel mit einer Parfümkomponente und eine waschaktive Tensidkomponente enthalten, dadurch gekennzeichnet, dass die Matrixpartikel aus einem gelförmigen Polymer aus Glucose, Glucuronsäure und Rhamnose bestehen.

Dem erfindungsgemäßen Pflegemittel genügt eine geringe Krafteinwirkung zu einer Freisetzung der Parfümkomponente und gegebenenfalls weiterer Inhaltsstoffe. Ein weiterer unerwarteter Vorteil der erfindungsgemäßen Matrixpartikel ist, dass sie im Gegensatz zu Koazervaten auch mit festen bzw. wasserlöslichen Bestandteilen beladen werden können. Bei Koazervaten ist dies bedingt durch den Ausfüllungsprozess nicht möglich.

Matrixpartikel für die erfindungsgemäßen Pflegemittel sind an sich bekannt (WO 98/15192, Gellan Gum, A Multi-functional Polysaccharide for Gelling and Texturising, Firmenschrift Monsanto von 1992, Technical Bulletin" The Preparation of Kelcogel® Gellan Gum Products" Firmenschrift von The Nutra Sweet Kelco Company RC-137).

Bei den Matrixpartikeln für die erfindungsgemäßen Pflegemittel handelt es sich um multifunktionelle Polysaccharide auf Basis von Stärke, Guaran, Johannisbrotkernmehl, Traganteinheiten, Xanthangummi, Gummiarabikum, Carboxymethylcellulose, Alginaten, Methylcellulose und Karaya-Gummi. Besonders bevorzugt sind Polysaccharide, die aus Glucose, Glucuronsäure und Rhamnose aufgebaut sind. Bevorzugt ist hierbei ein Verhältnis dieser Bausteine von 2:1:1.

Besonders bevorzugte Matrixpartikel für die erfindungsgemäßen Pflegemittel bestehen aus Gellan Gum. Gellan Gum ist ein wasserlösliches Polysaccharid, welches durch aerobe Fermentation von Pseudomonas eludea gewonnen wird. Die Mikroorganismen werden von einem Nährmedium mit einer Kohlenstoffquellen, Phosphaten, organischen und anorganischen Stickstoffverbindungen sowie Spurenelementen versorgt. Für die Wachstumsbedingungen sind sterile Arbeitsweisen, Sauerstoffzufuhr, Bewegung, Temperatur- und p_{H}-Wert-Kontrolle Voraussetzung. Anschließend wird die Fermentationsbrühe pasteurisiert um die lebenden Zellen abzutöten. Aus der Fermentationsbrühe wird Gellan Gum gewonnen.

Bei der Anlagerung von Salze an die Carboxylgruppen findet Aggregation des Matrixpartikels statt, wobei die Röntgenbeugungsanalyse zeigt, dass die Matrixpartikel den Aufbau einer links drehenden parallelen Doppelhelix aufweisen, die dreifach gefaltet ist.

Matrixpartikel mit einer Parfümkomponente können beispielsweise hergestellt werden, indem ein multifunktionelles Polysaccharid bei geeigneter Hydratationstemperatur in Wasser aufgelöst, die Parfümkomponente zugegeben und eine Emulsion hergestellt wird, wobei die Emulsion tropfenweise in eine wäßrige Lösung von Salze gegeben wird.

Polysaccharid und Parfümkomponente werden im allgemeinen im Verhältnis 1 zu 30, bevorzugt 1 zu 20, eingesetzt.

Salze sind bevorzugt Calziumchlorid, Kaliumchlorid, Magnesiumchlorid und Natriumchlorid.

Im allgemeinen enthält die wäßrige Lösung 0,001 bis 2 Gew.Teile, bevorzugt 0,01 bis 0,5 Gew.Teile an Salze.

Die Größe der Matrixpartikel kann durch die Größe der Eintropfkapillare gesteuert werden.

Die erfindungsgemäßen Pflegemittel enthalten im allgemeinen 0,1 bis 20 Gewichtsteile, bevorzugt 5 bis 15 Gewichtsteile, insbesondere bevorzugt 8 bis 12 Gewichtsteile an Matrixpartikel.

Die Parfümkomponente kann aus einem oder mehreren, bevorzugt 20 bis 60 Riechstoffen bestehen.

Die Parfümkomponente ist im wesentlichen in Wasser unlöslich.

Beispiele für Riechstoffe für die erfindungsgemäßen Pflegemittel finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3rd. Ed., Wiley-VCH, Weinheim 1997.

### Als Riechstoffe seien beispielsweise genannt:

Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedemblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;

Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinoat; Methyl-2-noninoat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavandulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpineol-4; Menthan-8-ol; Menthan-1-ol; Menthan-7-ol; Borneol; Isobomeol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedemholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha-3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentylteirahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenyl-glycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis-und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die erfindungsgemäßen Pflegemittel enthalten im allgemeinen 0,1 bis 20 Gewichtsteile, bevorzugt 0,5 bis 2 Gewichtsteile, insbesondere bevorzugt 0,8 bis 1,2 Gewichtsteile, an der Parfümkomponente.

Die erfindungsgemäßen Pflegemittel enthalten außerdem eine waschaktive Tensidkomponente. Die waschaktive Tensidkomponente kann aus einem oder mehreren, bevorzugt 3 bis 5, Tensiden bestehen.

Waschaktive Tenside für die erfindungsgemäßen Pflegemittel sind an sich bekannt (Andreas Domsch, Die kosmetischen Präparate, Band II).

Beispielsweise seien die folgenden waschaktiven Tenside genannt:

### Anionische Tenside

Zu dieser Stoffklasse zählen sekundäre Alkansulfonate z.B. Sodium C₁₄₋₁₇ Alkyl Sec Sulfonate, Alkylsulfate z.B. Sodium Lauryl Sulfate, Alkylethersulfate, Laurylethersulfate z.B. Sodium Laureth Sulfate, Olefinsulfonate z.B. Sodium Olefin C₁₄₋₁₆ Sulfonate, Alkylamidethersulfate z.B. TEA-PEG-3 Cocamide Sulfate, Acylisethionate z.B. Sodium Cocoyl Isethionate, Acylglutamate z.B. Lauroyl Glutamate, Alkylethercarboxylate z.B. Trideceth-7 Carboxylic Acid, Methyltauride und Tauride z.B. Sodium Lauroyl Taurate, Sarkoside z.B. Ammonium Cocoyl Sarcosinate, Sulfosuccinate z.B. Disodium PEG-4 Cocamido MIPA-Sulfosuccinate, Eiweißfettsäurekondensate z.B. Potassium Cocoyl Hydrolyzed Collagen.

### Amphotere Tenside

Zu dieser Stoffklasse zählen Alkylbetaine z.B. Lauryl Betaine, Alkylamidobetaine z.B. Cocamidopropyl Betaine, Sulfobetaine z.B. Cocamidopropyl Hydroxysultaine, Acetate und Diacetate z.B. Disodium Cocoamphodiacetate, Imidazoline z.B. Amphonyl, Propionate z.B. Sodium Cocoamphopropionate.

### Nichtionische Tenside

Zu dieser Stoffklasse zählen ethoxylierte Ricinusölderivate z.B. PEG-20 Hydrogenated Castor Oil, Polysorbate z.B. Polysorbate 80, Glycerinfettsäureester-Ethoxylate z.B. PEG-20 Glyceryl Isostearate, Zuckertenside z.B. Capryl/Caprylylglucoside, Aminoxide z.B. Cocamine Oxide.

Die erfindungsgemäßen Pflegemittel enthalten im allgemeinen 0,1 bis 50 Gewichtsteile, bevorzugt 1 bis 20 Gewichtsteile, insbesondere bevorzugt 5 bis 10 Gewichtsteile der waschaktiven Tensidkomponente.

Als weitere Komponente enthalten die erfindungsgemäßen Pflegemittel Wasser.

Die erfindungsgemäßen Pflegemittel können außerdem noch Elektrolyte enthalten.

Als Elektrolyte für die erfindungsgemäßen Pflegemittel seien beispielsweise genannt:

Natriumchlorid, Ammoniuchlorid und Magnesiumchlorid.

Die erfindungsgemäßen Pflegemittel können im allgemeinen 0,1 bis 5 Gewichtsteile, bevorzugt 0,3 bis 3 Gewichtsteile, insbesondere bevorzugt 0,5 bis 2 Gewichtsteile an Elektrolyte enthalten.

Die erfindungsgemäßen Pflegemittel können wie folgt hergestellt werden:

Das Gellan Gum wird in Wasser bei etwa 70°C gelöst, anschließend abgekühlt auf etwa 55°C und die Parfümkomponente zugegeben. Die Lösung wird mit einem Hochgeschwindigkeitsrührer homogenisiert und unter Rühren auf etwa 45°C abgekühlt. Gerührt wird die Lösung während der gesamten Herstellung. Mit einer Dosierpumpe wird die Lösung in ein eisgekühltes, wässriges Kalziumbad mit einer Konzentration von etwa 6 mmol Kalizumion gepumpt.

Die gewaschenen Matrixpartikel werden zu der Tensidformulierung gegeben.

Die nach diesem Verfahren hergestellten Pflegemittel bestehen aus Matrixpartikeln mit Kugeldurchmesser von 3 bis 5 mm. Die Form der Partikel ist gleichmäßig rund. Die Partikel haben einen mittleren Härtegrad und lassen sich ohne gezielte mechanische Einwirkung, wie sie z.B. beim Dusch- oder Waschvorgang vorkommt, zerstören.

Die erfindungsgemäßen Pflegemittel können im Bereich der Körperreinigung und bei technischen Reinigungsmitteln, z.B. Haushaltsreiniger, verwendet werden.

Überraschenderweise weisen die erfindungsgemäßen Pflegemittel auf Basis der Matrixpartikel ein hohes Retentionsverhalten für Tensidkomponenten, Parfümöle und andere Wirkstoffe auf.

Es ist leicht möglich, den Matrixpartikeln auch feste Komponenten, beispielsweise Farbpigmente, zuzufügen.

Ein Vorteil der erfindungsgemäßen Pflegemittel auf Basis von Matrixpartikel liegt in ihrem einfachen Rezepturaufbau und einem unkomplizierten Herstellungsverfahren. Der geringe apparative Aufbau ist somit leicht auf den Produktionsmaßstab zu übertragen.

Die Erfindung zeigt die folgenden weiteren unerwarteten Vorteile:
- Pflegemittel mit den erfindungsgemäßen Matrixpartikeln neigen nicht zum "Ausbluten" der Füllguts
- Pflegemittel auf Basis der Matrixpartikel weisen eine hohe geruchliche Stabilität in tensidischen Systemen auf.
- Pflegemittel auf Basis der Matrixpartikel haben eine hohe chemische Stabilität und Haftwirkung.
- Die erfmdungsgemäßen Pflegemittel auf Basis von Matrixpartikel ermöglichen einen gezielten Einsatz der Tensid- und Parfümkomponenten und belasten durch hohe Dosierung nicht die Umwelt.

### Beispiele

### 1. Shower Gel mit Matrixpartikel und Carbopol zum Schutz vor Sedimentation oder Aufrahmung:

| | %-Gehalt |
|---|---|
| Wasser | 42,9 |
| Carbopol ETD 2020 | 1,0 |
| Propylene Glykol | 5,0 |
| Ethanol | 5,0 |
| Triethanolamin | 1,0 |
| TEA-Lauryl-Sulfate | 30,0 |
| Konservierungsmittel | 0,1 |
| Texapon SB 3 KC | 2,0 |
| Lauramide DEA | 2,0 |
| Matrixpartikel mit 10 % Parfüm | 10,0 |

### 2. Schäumendes Duschbad:

| | %-Gehalt |
|---|---|
| Wasser | 67,3 |
| Plantacare PS 10 | 8,7 |
| Texapon ASV | 5,7 |
| Tego Betain L7 | 5,7 |
| Cetiol HE | 1,0 |
| Konservierungsmittel | 0,5 |
| Polymer JR 400 | 0,1 |
| Keltrol BT | 1,0 |
| Matrixpartikel mit 10 % Parfüm | 10,0 |

### 3. Geschirrspülmittel

| | %-Gehalt |
|---|---|
| Marlon A360 | 25,0 |
| Marlipal O13/90 | 4,0 |
| Serdolamide PPF 67 | 1,0 |
| Ethanol | 3,0 |
| Harnstoff | 2,0 |
| Keltrol BT | 1,0 |
| Matrixpartikel mit 2 % Parfüm | 10,0 |
| Wasser, Konservierungsmittel | 54,0 |

## Patentansprüche

1. Pflegemittel, enthaltend Matrixpartikel mit einer Parfümkomponente und einer waschaktive Tensidkomponente, **dadurch gekennzeichnet, dass** die Matrixpartikel aus einem gelförmigen Polymer aus Glucose, Glucuronsäure und Rhamnose bestehen.

2. Pflegemittel nach Anspruch 1, enthaltend 0,1 bis 50 Gew.-Teile einer waschaktiven Tensidkomponente.

3. Pflegemittel nach den Ansprüchen 1 und 2, enthaltend 1 bis 20 Gew.-Teile einer waschaktiven Tensidkomponente.

4. Pflegemittel nach den Ansprüchen 1 bis 3, enthaltend 5 bis 10 Gew.-Teile einer waschaktiven Tensidkomponente.

5. Pflegemittel nach den Ansprüchen 1 bis 4, enthaltend 0,1 bis 20 Gew.-Teile an Matrixpartikel.

6. Pflegemittel nach den Ansprüchen 1 bis 5, enthaltend 5 bis 15 Gew.-Teile an Matrixpartikel.

7. Pflegemittel nach den Ansprüchen 1 bis 6, enthaltend 8 bis 12 Gew.-Teile an Matrixpartikel.

8. Pflegemittel nach den Ansprüchen 1 bis 7, enthaltend 60 bis 99 Gew.-Teile an Wasser.

9. Pflegemittel nach den Ansprüchen 1 bis 8, enthaltend 65 bis 95 Gew.-Teile an Wasser.

10. Pflegemittel nach den Ansprüchen 1 bis 9, enthaltend 80 bis 90 Gew.-Teile an Wasser.

11. Pflegemittel nach den Ansprüchen 1 bis 10, enthaltend eine Parfümkomponente.

12. Pflegemittel nach den Ansprüchen 1 bis 11, enthaltend 0,1 bis 20 Gew.-Teile an Parfümkomponente.

13. Pflegemittel nach den Ansprüchen 1 bis 12, enthaltend 0,3 bis 5 Gew.-Teile an Parfümkomponente.

14. Pflegemittel nach den Ansprüchen 1 bis 13, enthaltend 0,5 bis 2 Gew.-Teile an Parfümkomponente.

15. Pflegemittel nach den Ansprüchen 1 bis 14, enthaltend 0,1 bis 10 Gew.-Teile eines Elektrolyten.

16. Pflegemittel nach den Ansprüchen 1 bis 15, enthaltend 0,2 bis 5 Gew.-Teile eines Elektrolyten.

17. Pflegemittel nach den Ansprüchen 1 bis 16, enthaltend 0,3 bis 3 Gew.-Teile eines Elektrolyten.

18. Pflegemittel nach den Ansprüchen 1 bis 17, **dadurch gekennzeichnet, dass** die Matrixpartikel einen Durchmesser von 0,01 bis 20 mm haben.

19. Pflegemittel nach den Ansprüchen 1 bis 18, **dadurch gekennzeichnet, dass** die Matrixpartikel einen Durchmesser von 0,1 bis 10 mm haben.

20. Pflegemittel nach den Ansprüchen 1 bis 19, **dadurch gekennzeichnet, dass** die Matrixpartikel aus gelförmigen Polysacchariden bestehen.

21. Pflegemittel nach den Ansprüchen 1 bis 20, **dadurch gekennzeichnet, dass** die Matrixpartikel aus einem gelförmigen Polymer aus Glucose, Glucoronsäure und Rhamnose im Verhältnis 2:1:1 bestehen.

22. Pflegemittel nach den Ansprüchen 1 bis 21, **dadurch gekennzeichnet, dass** die Matrixpartikel Füllstoffe, kosmetische Additive, Farbstoffe oder Wirkstoffe enthalten.

23. Verwendung von Pflegemitteln nach den Ansprüchen 1 bis 22 in Körperreinigungsmittel.

24. Verwendung von Pflegemitteln nach den Ansprüchen 1 bis 22 in Haushaltsreiniger.

## Claims

1. Care agents comprising matrix particles having a perfume component and a washing-active tenside component, **characterized in that** the matrix particles consist of a gelatinous polymer of glucose, glucuronic acid and rhamnose.

2. Care agents according to Claim 1, comprising 0.1 to 50 parts by weight of a washing-active tenside component.

3. Care agents according to Claims 1 and 2, comprising 1 to 20 parts by weight of a washing-active tenside component.

4. Care agents according to Claims 1 to 3, comprising 5 to 10 parts by weight of a washing-active tenside component.

5. Care agents according to Claims 1 to 4, comprising 0.1 to 20 parts by weight of matrix particles.

6. Care agents according to Claims 1 to 5, comprising 5 to 15 parts by weight of matrix particles.

7. Care agents according to Claims 1 to 6, comprising 8 to 12 parts by weight of matrix particles.

8. Care agents according to Claims 1 to 7, comprising 60 to 99 parts by weight of water.

9. Care agents according to Claims 1 to 8, comprising 65 to 95 parts by weight of water.

10. Care agents according to Claims 1 to 9, comprising 80 to 90 parts by weight of water.

11. Care agents according to Claims 1 to 10, comprising a perfume component.

12. Care agents according to Claims 1 to 11, comprising 0.1 to 20 parts by weight of perfume component.

13. Care agents according to Claims 1 to 12, comprising 0.3 to 5 parts by weight of perfume component.

14. Care agents according to Claims 1 to 13, comprising 0.5 to 2 parts by weight of perfume component.

15. Care agents according to Claims 1 to 14, comprising 0.1 to 10 parts by weight of an electrolyte.

16. Care agents according to Claims 1 to 15, comprising 0.2 to 5 parts by weight of an electrolyte.

17. Care agents according to Claims 1 to 16, comprising 0.3 to 3 parts by weight of an electrolyte.

18. Care agents according to Claims 1 to 17, **characterized in that** the matrix particles have a diameter of from 0.01 to 20 mm.

19. Care agents according to Claims 1 to 18, **characterized in that** the matrix particles have a diameter of from 0.1 to 10 mm,

20. Care agents according to Claims 1 to 19, **characterized in that** the matrix particles consist of gelatinous polysaccharides.

21. Care agents according to Claims 1 to 20, **characterized in that** the matrix particles consist of a gelatinous polymer of glucose, glucuronic acid and rhamnose in the ratio 2:1:1.

22. Care agents according to Claims 1 to 21, **characterized in that** the matrix particles comprise fillers, cosmetic additives, dyes or active ingredients.

23. Use of care agents according to Claims 1 to 22 in body-cleansing compositions.

24. Use of care agents according to Claims 1 to 22 in household cleaners.

## Revendications

1. Produit de soins, contenant des particules de matrice avec un composant de parfum et un composant tensioactif détergent, **caractérisé en ce que** les particules de matrice sont composées d'un polymère sous forme de gel contenant du glucose, des acides glucuroniques et du rhamnose.

2. Produit de soins selon la revendication 1, contenant 0,1 à 50 parties en points d'un composant tensioactif détergent.

3. Produit de soins selon les revendications 1 et 2, contenant 1 à 20 parties en poids d'un composant tensioactif détergent.

4. Produit de soins selon les revendications 1 à 3, contenant 5 à 10 parties en poids d'un composant tensioactif détergent.

5. Produit de soins selon les revendications 1 à 4, contenant 0,1 à 20 parties en poids de particules de matrice.

6. Produit de soins selon les revendications 1 à 5, contenant 5 à 15 parties en poids de particules de matrice.

7. Produit de soins selon les revendications 1 à 6, contenant 8 à 12 parties en poids de particules de matrice.

8. Produit de soins selon les revendications 1 à 7, contenant 60 à 99 parties en poids d'eau.

9. Produit de soins selon les revendications 1 à 8, contenant 65 à 95 parties en poids d'eau.

10. Produit de soins selon les revendications 1 à 9, contenant 80 à 90 parties en poids d'eau.

11. Produit de soins selon les revendications 1 à 10, contenant un composant de parfum.

12. Produit de soins selon les revendications 1 à 11, contenant 0,1 à 20 parties en poids d'un composant de parfum.

13. Produit de soins selon les revendications 1 à 12, contenant 0,3 à 5 parties en poids d'un composant de parfum.

14. Produit de soins selon les revendications 1 à 13, contenant 0,5 à 2 parties en poids d'un composant de parfum.

15. Produit de soins selon les revendications 1 à 14, contenant 0,1 à 10 parties en poids d'un électrolyte.

16. Produit de soins selon les revendications 1 à 15, contenant 0,2 à 5 parties en poids d'un électrolyte.

17. Produit de soins selon les revendications 1 à 16, contenant 0,3 à 3 parties en poids d'un électrolyte.

18. Produit de soins selon les revendications 1 à 17, **caractérisé en ce que** les particules de matrice ont un diamètre compris entre 0,01 et et 20 mm.

19. Produit de soins selon les revendications 1 à 18, **caractérisé en ce que** les particules de matrice ont un diamètre compris entre 0,1 et 10 mm.

20. Produit de soins selon les revendications 1 à 19, **caractérisé en ce que** les particules de matrice sont composées de polysaccharides sous forme de gel.

21. Produit de soins selon les revendications 1 à 20, **caractérisé en ce que** les particules de matrice sont composées d'un polymère sous forme de gel contenant du glucose, de l'acide glucuronique et du rhamnose, dans un rapport de 2 :1 :1.

22. Produit de soins selon les revendications 1 à 21, **caractérisé en ce que** les particules de matrice contiennent des charges, des additifs cosmétiques, des colorants et des substances actives.

23. Utilisation de produits de soins selon les revendications 1 à 22 dans des produits de nettoyage du corps.

24. Utilisation de produits de soins selon les revendications 1 à 22 dans des produits d'entretien ménagers.
